# EUROPEAN PATENT APPLICATION

(11) **EP 4 190 279 A1**
(43) Date of publication of application: **07.06.2023**
(21) Application number: 21863224.8
(22) Date of filing: 12.04.2021
(51) Int. Cl.: A61F 2/24

(54) **INTERVENTIONAL VALVE STENT AND AORTIC VALVE**

(30) Priority: 04.09.2020 CN 202010924332
(71) Applicant: Shanghai Newmed Medical Co., Ltd., Shanghai 201321 (CN)
(72) Inventor: ZHAN, Hangmin, Shanghai 201321 (CN); YU, Qifeng, Shanghai 201321 (CN); QIN, Tao, Shanghai 201321 (CN)
(74) Representative: Wang, Bo
(86) International application number: PCT/CN2021/086495
(87) International publication number: WO 2022/048154

(57) **Abstract**

An interventional valve stent (100) and an aortic valve, wherein the interventional valve stent (100) includes: a valve stent defining a frame lumen, the valve stent includes straight rods (1) connecting an upstream port (A) and a downstream port (B), and oblique rods (2) connected between the straight rods (1); an upstream section (10), a midstream section (20), and a downstream section (30) are sequentially formed along a direction from the upstream port (A) to the downstream port (B), when the valve stent expands from a compressed state to an expanded state, an expansion strain provided by the oblique rods (2) located in the midstream section (20) to a circumferential direction of the valve stent is greater than an expansion strain provided by the oblique rods (2) located in the upstream section (10) and/or the downstream section (30) to the circumferential direction of the valve stent to compensate for a rate difference between a rate of circumferential expansion of the midstream section (20) and a rate of circumferential expansion of the upstream section (10) and/or a rate of circumferential expansion of the downstream section (30). The interventional valve stent (100) can effectively accept the stress transmitted from the oblique rods (2) in the midstream section (20), the stress concentration of the oblique rods (2) in the midstream section (20) can be reduced, so that the oblique rods (2) in the midstream section (20) is more likely to be expanded under force, and the expansion rate of the oblique rods (2) at both two ends can be achieved, thereby reducing or alleviating the adverse effect of the expansion of the two ends of the valve stent to form a dog-bone structure.

## Description

### TECHNICAL FIELD

The present disclosure generally relates to the field of minimally invasive medical devices, and in particular, relates to an interventional valve stent and an aortic valve.

### BACKGROUND

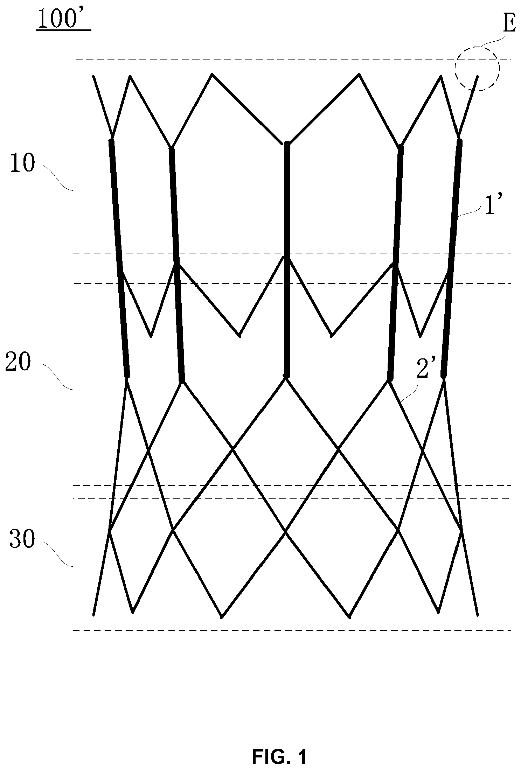

FIG. 1 is a schematic perspective view of an interventional valve stent in prior arts. As shown in FIG. 1, an existing interventional valve stent 100' includes a straight rod 1 and an oblique rod 2. During an expansion process, since an upstream section 10, a midstream section 20 and a downstream section 30 of the interventional valve stent 100' are in different positions, a dog bone effect may appear. That is, when a valve stent delivery system is filled to the maximum recommended filling pressure, diameters of a proximal end and a distal end of the valve stent are greater than a diameter of a middle end of the valve stent, and an appearance of the valve stent may look like a dog bone. This is because during the expansion process of the valve stent, an expansion rate of the midstream section 20 is significantly lower than an expansion rate of the upstream section 10 and an expansion rate of the downstream section 30. It is very dangerous to encounter this phenomenon clinically, because sharps (e.g., an E area) at two ends of the expanded valve stent may cause unnecessary damage to aortic tissue. At the same time, an actual diameter of the midstream section may be less than a preset diameter, resulting in loosening or even slippage of the valve stent on the aortic sinus ring.

### SUMMARY

In view of problems in prior arts, the purpose of the present disclosure is to provide an interventional valve stent and an aortic valve, which overcomes the problem that the two ends of the valve stent in prior arts are easy to damage the tissue when the valve stent expands. The stress concentration of the oblique rods in the midstream section may be reduced by effectively accepting the stress transmitted by the oblique rods in the midstream section. Thus, the oblique rods in the midstream section may be more likely to expand under stress, to reach the expansion rate of the oblique rods at two ends, thereby reducing or alleviating an adverse effect of the expansion of the two ends of the valve stent to form a dog-bone structure.

Embodiments of the present disclosure provides an interventional valve stent. The interventional valve stent may include a valve stent defining a frame lumen. The valve stent may include straight rods connecting an upstream port and a downstream port, and oblique rods connected between the straight rods. An upstream section, a midstream section, and a downstream section may be sequentially formed along a direction from the upstream port to the downstream port. When the valve stent expands from a compressed state to an expanded state, an expansion strain provided by the oblique rods located in the midstream section to a circumferential direction of the valve stent may be greater than an expansion strain provided by the oblique rods located in the upstream section and/or the downstream section to the circumferential direction of the valve stent to compensate for a rate difference between a rate of circumferential expansion of the midstream section and a rate of circumferential expansion of the upstream section and/or a rate of circumferential expansion of the downstream section.

In some embodiments, during an expansion process of the valve stent, the rate of circumferential expansion of the midstream section may be the same as the rate of circumferential expansion of the upstream section and/or the rate of circumferential expansion of the downstream section.

In some embodiments, the straight rods may be distributed in parallel in a column direction, and the oblique rods may be distributed between the straight rods in multiple rows along a row direction perpendicular to the column direction.

In some embodiments, from a middle of the valve stent to two ends of the valve stent, rod widths of the oblique rods located in different rows may increase sequentially.

In some embodiments, from the middle of the valve stent to the two ends of the valve stent, the rod widths of the oblique rods located in different rows may increase linearly or in stages in an order of rows.

In some embodiments, from a middle of the valve stent to two ends of the valve stent, wall thicknesses of the oblique rods located in different rows may increase sequentially.

In some embodiments, from the middle of the valve stent to the two ends of the valve stent, the wall thicknesses of the oblique rods located in different rows may increase linearly or in stages in an order of rows.

In some embodiments, the oblique rods may be connected in a V-shaped arrangement between two adjacent straight rods to form an included angle between rods. From a middle of the valve stent to two ends of the valve stent, included angles between rods of the oblique rods located in different rows may increase sequentially.

In some embodiments, from the middle of the valve stent to the two ends of the valve stent, the included angles between rods of the oblique rods located in different rows may increase linearly or in stages in an order of rows.

Embodiments of the present disclosure provides an interventional valve stent. The interventional valve stent may include a valve stent defining a frame lumen. The valve stent may include straight rods connecting an upstream port and a downstream port, and oblique rods connected between the straight rods. An upstream section, a midstream section, and a downstream section may be sequentially formed along a direction from the upstream port to the downstream port. When the valve stent expands from a compressed state to an expanded state, an expansion strain provided by the oblique rods located in the midstream section to a circumferential direction of the valve stent may be greater than an expansion strain provided by the oblique rods located in the upstream section and/or the downstream section to the circumferential direction of the valve stent to compensate for a rate difference between a rate of circumferential expansion of the midstream section and a rate of circumferential expansion of the upstream section and/or a rate of circumferential expansion of the downstream section. The straight rods may be distributed in parallel in a column direction, and the oblique rods may be distributed between the straight rods in multiple rows along a row direction perpendicular to the column direction. The oblique rods may be connected in a V-shaped arrangement between two adjacent straight rods to form an included angle between rods. The oblique rods may satisfy a requirement: from a middle of the valve stent to two ends of the valve stent, rod widths, wall thicknesses, and included angles between rods of the oblique rods located in different rows all increase sequentially.

Embodiments of the present disclosure provides an aortic valve. The aortic valve may include an interventional valve stent as described above, and a valve leaflet arranged in a frame lumen formed by the valve stent.

The interventional valve stent and the aortic valve of the present disclosure are conducive to enhancing the internal stress transmission of the oblique rods in the midstream section during the expansion of the valve stent. The stress concentration of the oblique rods in the midstream section may be reduced by effectively accepting the stress transmitted by the oblique rods in the midstream section. Thus, the oblique rods in the midstream section may be more likely to expand under stress, to reach the expansion rate of the oblique rods at two ends, thereby reducing or alleviating the adverse effect of the expansion of the two ends of the valve stent to form a dog-bone structure.

### BRIEF DESCRIPTION OF THE DRAWINGS

Other features, objectives, and advantages of the present disclosure may become more apparent by reading the detailed description of non-limiting embodiments with reference to the following drawings.
FIG. 1 is a schematic perspective view illustrating an interventional valve stent in prior arts;
FIG. 2 is a schematic perspective view illustrating a first type of interventional valve stent of the present disclosure;
FIG. 3 is a flattened plan view illustrating the first type of interventional valve stent of the present disclosure;
FIG. 4 is a flattened plan view illustrating a second type of interventional valve stent of the present disclosure;
FIG. 5 is a flattened plan view illustrating a third type of interventional valve stent of the present disclosure;
FIG. 6 is a flattened plan view illustrating a fourth type of interventional valve stent of the present disclosure;
FIG. 7 is a flattened plan view illustrating a fifth type of interventional valve stent of the present disclosure; and
FIG. 8 is a flattened plan view illustrating a sixth type of interventional valve stent of the present disclosure.

### DETAILED DESCRIPTION

Example embodiments may be described more comprehensively with reference to the accompanying drawings. However, the example embodiments can be implemented in many forms and should not be understood as limited to the embodiments described herein. On the contrary, these embodiments are provided to make the present disclosure comprehensive and complete, and the concept of the example embodiments can be conveyed to those skilled in the art comprehensively. The same reference numerals in the drawings denote the same or similar structures, and thus the repeated descriptions may be omitted.

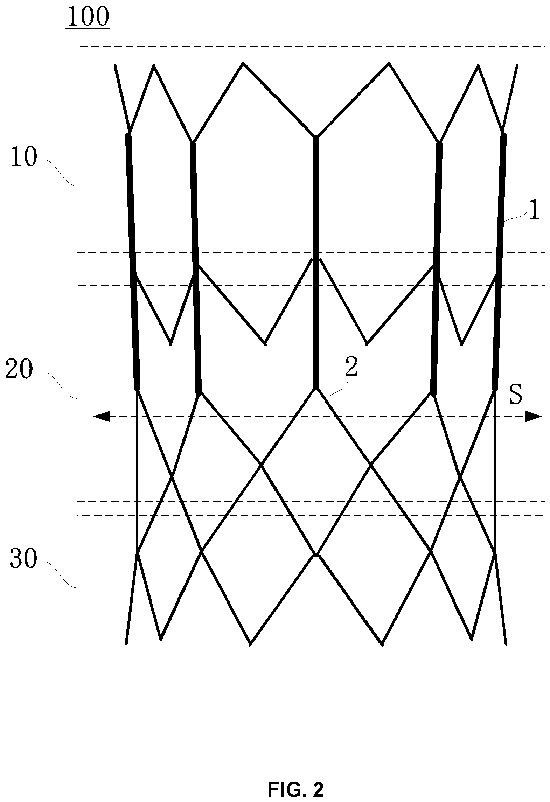

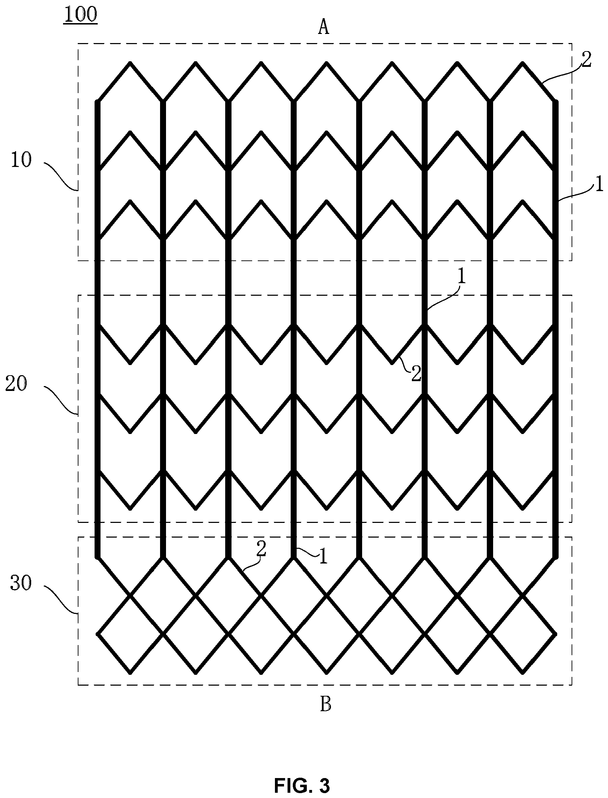

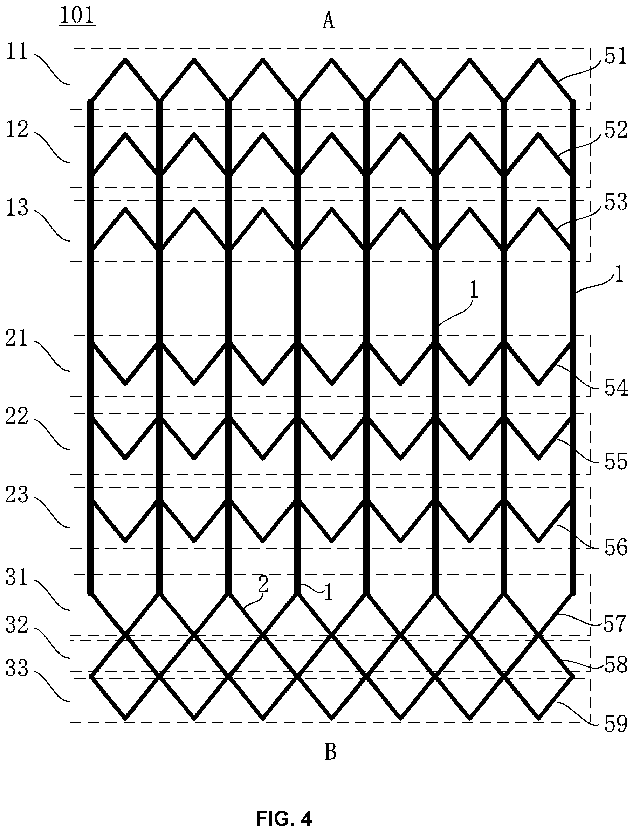

FIG. 2 is a schematic perspective view illustrating a first type of interventional valve stent of the present disclosure. FIG. 3 is a flattened plan view illustrating the first type of interventional valve stent of the present disclosure. As shown in FIGs 2 and 3 (in order to see an arrangement of each oblique rod in the interventional valve stent, FIG. 3 shows a schematic view of an interventional valve stent 100 after the interventional valve stent is flattened, which does not mean that the interventional valve stent is a planar product; similarly, subsequent FIGs 4, 5, 6, 7, and 8 are all schematic diagrams of interventional valve stents after the interventional valve stents are flattened), the interventional valve stent 100 of the present disclosure may include a valve stent defining a frame lumen. The valve stent may expand from a compressed state to an expanded state. The valve stent may include straight rods 1 connecting an upstream port A and a downstream port B, and oblique rods 2 connected between the straight rods 1. A tubular opening may be formed at the upstream port A, and a skirt opening may be formed at the downstream port B. In some embodiments, the straight rods 1 may be distributed in parallel in a column direction, and the oblique rods 2 may be distributed between the straight rods 1 in multiple rows along a row direction perpendicular to the column direction. The oblique rods 2 may be connected in a V-shaped arrangement between two adjacent straight rods 1. An upstream section 10, a midstream section 20, and a downstream section 30 may be sequentially formed along a direction from the upstream port A to the downstream port B. An expansion strain provided by the oblique rods 2 located in the midstream section 20 to a circumferential direction of the valve stent may be greater than an expansion strain provided by the oblique rods 2 located in the upstream section 10 and/or the downstream section 30 to the circumferential direction of the valve stent.

In the embodiment, the expansion strain provided by the oblique rods 2 to the circumferential direction of the valve stent may refer to an extent to which the oblique rods 2 in different rows in the valve stent expand outward to the circumferential direction of the valve stent after being strained, wherein compared with the oblique rods 2 in the upstream section 10 and the oblique rods 2 in the downstream section 30, the oblique rods 2 in the midstream section 20 may be more likely to expand outward after being stressed, such that, compared with prior arts, the midstream section 20 is easier to bulge out in a S direction (circumferential bulge out at the same time). Subject to the mutual pull and limitation between the oblique rods 2 and the straight rods 1, the sharps at the two ends of the interventional valve stent 100 may no longer open outward, thereby reducing or alleviating the adverse effect of the outward expansion of the two ends of the valve stent to form a dog-bone structure.

In one embodiment, the expansion strain provided by the oblique rods 2 located in the midstream section 20 to the circumferential direction of the valve stent may only be greater than the expansion strain provided by the oblique rods 2 located in the upstream section 10 to the circumferential direction of the valve stent.

In one embodiment, the expansion strain provided by the oblique rods 2 located in the midstream section 20 to the circumferential direction of the valve stent may only be greater than the expansion strain provided by the oblique rods 2 located in the downstream section 30 to the circumferential direction of the valve stent.

In one embodiment, the expansion strain provided by the oblique rods 2 located in the midstream section 20 to the circumferential direction of the valve stent may be greater than the expansion strain provided by the oblique rods 2 located in the upstream section 10 to the circumferential direction of the valve stent, and may also be greater than the expansion strain provided by the oblique rods 2 in the downstream section 30 to the circumferential direction of the valve stent.

In the present disclosure, the oblique rods may satisfy at least one or a combination of two or more of the following conditions.

From a middle of the valve stent to two ends of the valve stent, rod widths of the oblique rods 2 located in different rows may increase sequentially. In the embodiment, the rod width of the oblique rod 2 may be a width dimension (a dimension in a width direction perpendicular to a length direction of the oblique rod 2) of a rectangular area where the oblique rod 2 is exposed on a surface of the frame lumen.

From the middle of the valve stent to the two ends of the valve stent, wall thicknesses of the oblique rods 2 located in different rows may increase sequentially. In the embodiment, the wall thickness of the oblique rod 2 may be a thickness dimension of the oblique rod 2 along a diameter direction of the lumen based on the frame lumen where it is located.

From the middle of the valve stent to the two ends of the valve stent, included angles between rods of the oblique rods located in different rows may increase sequentially. The included angle between rods may be an included angle formed by the two oblique rods 2 arranged in a V shape.

Through the above structures, when the valve stent is expanding, the oblique rods 2 in the midstream section 20 may provide a stronger expansion strain (stronger than the upstream section 10 and/or the downstream section 30) to the circumferential direction of the valve stent to compensate for a rate difference between a rate of circumferential expansion of the midstream section 20, and a rate of circumferential expansion of the upstream section 10 and/or the downstream section 30, such that the rate of circumferential expansion of the midstream section 20 is substantially the same as the rate of circumferential expansion of the upstream section 10 and/or the downstream section 30, but this is not limited. Thus, the diameters of the interventional valve stent in the upstream section 10, the midstream section 20, and the downstream section 30 may be substantially the same, avoiding the situation that the two ends of the expanded valve stent are sharp, avoiding unnecessary damage to the aortic tissue effectively, and preventing the valve stent from loosening or even slipping on the aortic sinus ring.

As shown in FIG. 3 , in a preferred embodiment, the extension strain provided by each row of oblique rods in the upstream section 10 to the circumferential direction of the valve stent may be equal. The expansion strain provided by each row of oblique rods in the middle section 20 to the circumferential direction of the valve stent may be equal. The expansion strain provided by each row of oblique rods in the downstream section 30 to the circumferential direction of the valve stent may be equal. The expansion strain provided by each row of oblique rods in the midstream section 20 to the circumferential direction of the valve stent may be greater than the expansion strain provided by each row of oblique rods in the upstream section 10 to the circumferential direction of the valve stent, and the expansion strain provided by each row of oblique rods in the midstream section 20 to the circumferential direction of the valve stent may be greater than the expansion strain provided by each row of oblique rods in the downstream section 30 to the circumferential direction of the valve stent. The difference of the expansion strain provided to the circumferential direction of the valve stent may be realized by the difference of the widths of the oblique rods 2, the wall thicknesses of the oblique rods 2, and the included angles between rods of the oblique rods 2, which may not be repeated here. Based on the above structure, the technical solutions for adjusting the expansion strain provided by the oblique rod 57 to the circumferential direction of the valve stent gradually and in stages fall within the scope of protection of the present disclosure.

FIG. 4 is a flattened plan view illustrating a second type of interventional valve stent of the present disclosure. As shown in FIG. 4, in an interventional valve stent 101 of the present disclosure, from an upstream port A to a downstream port B, oblique rods 51 in a first row 11, oblique rods 52 in a second row 12, oblique rods 53 in a third row 13, oblique rods 54 in a fourth row 21, oblique rods 55 in a fifth row 22, oblique rods 56 in a sixth row 23, oblique rods 57 in a seventh row 31, oblique rods 58 in an eighth row 32, and oblique rods 59 in a ninth row 33 may be sequentially formed. An extension strain provided by the oblique rods 55 located in the fifth row 22 to a circumferential direction of the valve stent may be greater than an expansion strain provided by the oblique rods 54 located in the fourth row 21 to the circumferential direction of the valve stent. The expansion strain provided by the oblique rods 54 located in the fourth row 21 to the circumferential direction of the valve stent may be greater than an expansion strain provided by the oblique rods 53 located in the third row 13 to the circumferential direction of the valve stent. The expansion strain provided by the oblique rods 53 in the third row 13 to the circumferential direction of the valve stent may be greater than an expansion strain provided by the oblique rods 52 in the second row 12 to the circumferential direction of the valve stent. The expansion strain provided by the oblique rods 52 in the second row 12 to the circumferential direction of the valve stent may be greater than an expansion strain provided by the oblique rods 51 in the first row 11 to the circumferential direction of the valve stent. Similarly, the expansion strain provided by the oblique rods 55 in the fifth row 22 to the circumferential direction of the valve stent may be greater than an expansion strain provided by the oblique rods 56 in the sixth row 23 to the circumferential direction of the valve stent. The expansion strain provided by the oblique rods 56 in the sixth row 23 to the circumferential direction of the valve stent may be greater than an expansion strain provided by the oblique rods 57 in the seventh row 31 to the circumferential direction of the valve stent. The expansion strain provided by the oblique rods 57 in the seventh row 31 to the circumferential direction of the valve stent may be greater than an expansion strain provided by the oblique rods 58 in the eighth row 32 to the circumferential direction of the valve stent. The expansion strain provided by the oblique rods 58 located in the eighth row 32 to the circumferential direction of the valve stent may be greater than an expansion strain provided by the oblique rods 59 located in the ninth row 33 to the circumferential direction of the valve stent. The difference in the expansion strain provided to the circumferential direction of the valve stent may be realized by the difference in the widths of the oblique rods 2, the wall thicknesses of the oblique rods 2, and the included angles between rods of the oblique rods 2, which may not be repeated here. In the embodiment, the oblique rods present a symmetrical distribution with the oblique rods 55 located in the fifth row 22 as a center line, and the closer each row of oblique rods is to the upstream port A or the downstream port B, the smaller the extended strain provided by the oblique rods to the circumferential direction of the valve stent. During the expansion process of the entire interventional valve stent 101, the oblique rods 51 located in the first row 11, the oblique rods 52 located in the second row 12, the oblique rods 53 located in the third row 13, the oblique rods 54 located in the fourth row 21, the oblique rods 55 located in the fifth row 22, the oblique rods 56 located in the sixth row 23, the oblique rods 57 located in the seventh row 31, the oblique rods 58 located in the eighth row 32, the oblique rods 59 located in the ninth row 33, there is a smooth transition of the strain from the oblique rod 55 in the middle to both sides, but it is not limited thereto. Based on the above structure, the technical solutions for adjusting the expansion strain provided by the oblique rod 57 to the circumferential direction of the valve stent gradually and in stages fall within the scope of protection of the present disclosure.

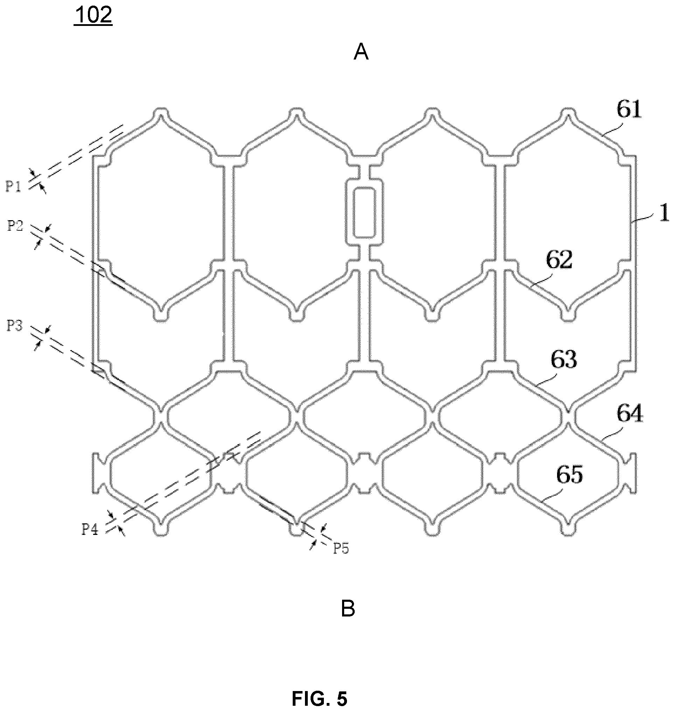

FIG. 5 is a flattened plan view illustrating a third type of interventional valve stent of the present disclosure. As shown in FIG. 5, in an interventional valve stent 102 of the present disclosure, from a middle of the valve stent to two ends of the valve stent, rod widths of oblique rods 2 located in different rows may increase linearly or in stages in an order of rows. The widths of the oblique rods in the middlemost row of the interventional valve stent 101 may be 0.36 mm, and the widths of the oblique rods in the most end rows may be 0.40 mm, but not limited thereto.

The interventional valve stent 102 in the present embodiment may be mainly supported by the oblique rods and the straight rods in structure. The oblique rods may be the main structural part that control the mechanical properties of the valve stent's compression and expansion. The oblique rods in the same row may have the same width and size. The oblique rods of the aortic valve stent may be divided into five rows according to the principle from top to bottom. The common rod type design may be that the widths of the rods in the five rows are the same. Such design may be relatively simple and direct, and the processing technology may be simple and convenient. However, since the oblique rods in the middle part have straight rods at the two ends to control a state of expansion movement, but the oblique rods at the two ends of the head and tail do not, the situation may lead to the dog bone effect.

As shown in FIG. 5, the interventional valve stent may include five rows of oblique rod 61, oblique rod 62, oblique rod 63, oblique rod 64, and oblique rod 65 arranged in parallel from an upstream port A to a downstream port B, wherein the rod width P1 of the oblique rod 61 may be 0.40 mm, the rod width P2 of the oblique rod 62 may be 0.38 mm, the rod width P3 of the oblique rod 63 may be 0.36 mm, the rod width P4 of the oblique rod 64 may be 0.38 mm, and the rod width P5 of the oblique rod 65 may be 0.40 mm .

In a preferred example, a change of rod width between rods in adjacent rows may be from 0.01 mm to 0.10 mm, but not limited thereto.

In a preferred example, the change of rod width between rods in adjacent rows may be 0.02 mm, but not limited thereto.

In a preferred example, the width of the oblique rod in the middle row of the interventional valve stent may be 0.26 mm, from the middle to both two ends of the interventional valve stent, a gradual change of rod width between adjacent oblique rods may be 0.02 mm, and the oblique rods in the rows at the two ends may be 0.29 mm, but not limited thereto.

The present disclosure, the rod widths of the oblique rods at the two ends may be widened, and the increase of the rod widths may cause the current expansion rate of the oblique rods to decrease, so that the expansion rate of the oblique rods at the two ends and the expansion rate of the oblique rods in the middle can be kept consistent, or appropriately slowed down, so as to eliminate or reduce the dog bone effect. During design, it is necessary to keep the gradual change of the rod width, that is, add a margin rod width of 2d at the two ends to an initial rod width, the two ends close to the middle end may be the initial rod width adds the margin rod width of d, and the middle rod width may be the initial design rod width. Thus, the change of rod width is not abrupt and the transition is smooth.

The following table takes the 26 mm aortic valve stent as an example for parallel comparison (comparison between all rod widths are 0.36 mm and a gradual change of rod width is 0.02 mm)

| Location | Consistent rod width (prior arts) | Gradual change of rod width (the present disclosure) |
|---|---|---|
| Inflow end diameter | 25.9 mm | 25.7 mm |
| Middle section | 24.2 mm | 25.0 mm |
| Outflow end | 25.7 mm | 25.5 mm |
| Deviation between inflow end and middle section | 1.7 mm | 0.7 mm |
| Deviation between outflow end and middle section | 1.5 mm | 0.5 mm |

From the comparison results in the above table, it can be seen that the structure of the gradual change of rod width of the interventional valve stent 102 in the present disclosure reduces the deviation between the inflow end and the middle section, and the deviation between the outflow end and the middle section, achieving the effect of reducing the dog bone effect.

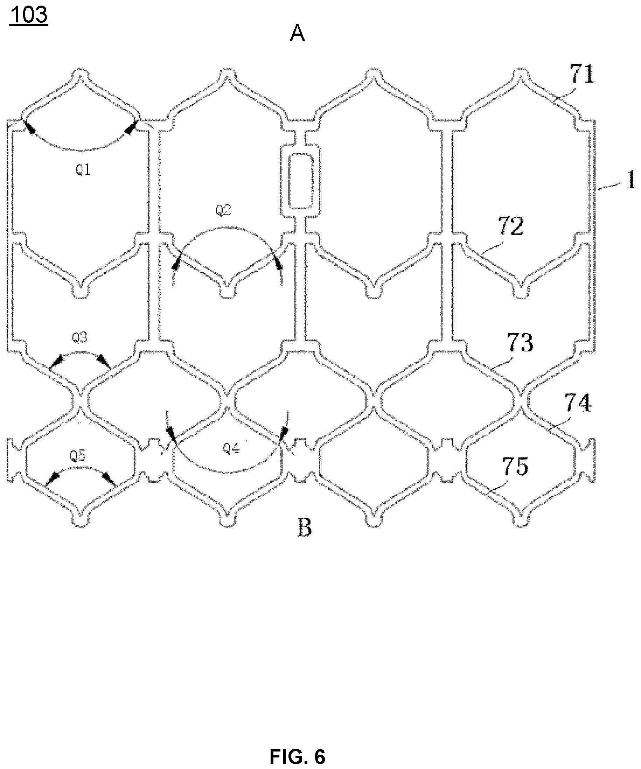

FIG. 6 is a flattened plan view illustrating a fourth type of interventional valve stent of the present disclosure. As shown in FIG. 6, similar to the design idea of the rod width, an interventional valve stent 103 may include five rows of oblique rod 71, oblique rod 72, oblique rod 73, oblique rod 74, and oblique rod 75 arranged in parallel along an upstream port A to a downstream port B. An included angle Q1 between rods of a first row of oblique rods 71 may be 120°, an included angle Q2 between rods of a second row of oblique rods 72 may be 113°, an included angle Q3 between rods of a third row of oblique rods 73 may be 106°, an included angle Q4 between rods of a fourth row of oblique rods 74 may be 113°, and an included angle Q5 between rods of the a fifth row of oblique rods 75 may be 120°.

A gradual change of included angle between rods may also properly eliminate the dog bone effect. As shown in FIG. 6, the included angle between rods may be appropriately increased at the two ends of the valve stent, and the included angle may gradually transit to an initial included angle θ in the middle of the valve stent. This design principle may be that the included angle between rods is almost 0 when the valve stent is in the crimped state, and the included angle between rods is gradually expanded to the design nominal included angle in the expanded state. The oblique rods at the two ends need to be deployed at relatively large included angles to reach a design outer diameter of the valve stent, while the oblique rods at the middle only need to be deployed at relatively small included angles to reach the design outer diameter.

In a preferred example, from the middle of the valve stent to the two ends of the valve stent, the included angles between rods of the oblique rods 2 in different rows may increase linearly and sequentially in an order of rows, but the present disclosure is not limited thereto.

In a preferred example, the included angle between rods of the oblique rods in the middle row of the aortic valve stent may be 120° (an angle range of the included angle between rods of the oblique rods in the middle row may be from 90° to 150°, preferably from 110° to 130°, and 120°may be the most preferred). From the middle to the two ends, a change of the included angle between rods between adjacent oblique rods may be from 2° to 20° (preferably from 4° to 10°, and from 6° to 7° may be the most preferred), but not limited to thereto.

The following table is verified by finite element analysis: take the 26 mm aortic valve stent as an example to verify the influence of the gradual change of included angle between rods on the dog bone effect (parallel comparison condition: comparison between all included angles between rods are 120 degrees and the gradual change of included angle between rods, the gradual change of included angle between rods is 7 degrees). The numerical comparison verification:

| Location | Included angle between rods is 120 degrees (prior arts) | Gradual change of included angle between rods (the present disclosure) |
|---|---|---|
| Inflow end diameter | 24.2 mm | 25.8 mm |
| Middle section | 22.0 mm | 24.5 mm |
| Outflow end | 24.6 mm | 25.7 mm |
| Deviation between inflow end and middle section | 2.2 mm | 1.3 mm |
| Deviation between outflow end and middle section | 2.6 mm | 1.2mm |

From the comparison results in the above table, it can be seen that the structure of the gradual change of included angle between rods of the interventional valve stent 103 in the present disclosure reduces the deviation between the inflow end and the middle section, and the deviation between the outflow end and the middle section, achieving the effect of reducing the dog bone effect.

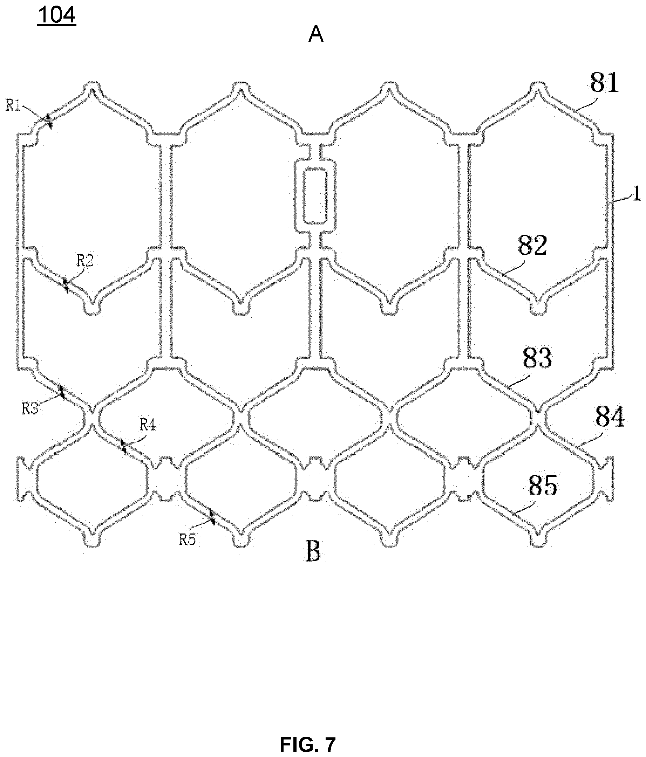

FIG. 7 is a flattened plan view illustrating a fifth type of interventional valve stent of the present disclosure. As shown in FIG. 7, an interventional valve stent 104 may include five rows of oblique rod 81, oblique rod 82, oblique rod 83, oblique rod 84, and oblique rod 85 arranged in parallel from an upstream port A to a downstream port B. A wall thickness R1 of the oblique rod 81 may be 0.50 mm, a wall thickness R2 of the oblique rod 82 may be 0.48 mm, a wall thickness R3 of the oblique rod 83 may be 0.46 mm, a wall thickness R4 of the oblique rod 84 may be 0.48 mm, a wall thickness R5 of the oblique rod 85 may be 0.50 mm. The interventional valve stent 104 may also achieve the technical effect that the expansion strain provided by the oblique rods 2 located in the midstream section 20 to the circumferential direction of the valve stent is greater than the expansion strain provided by the inclined rod 2 located in the upstream section 10 and/or downstream section 30 to the circumferential direction of the valve stent. By effectively accepting the stress transmitted by the oblique rods in the midstream section, the stress concentration of the oblique rods in the midstream section may be reduced, so that the oblique rods in the midstream section may be more likely to be expanded under force, and the expansion rate of the oblique rods at both two ends can be achieved, thereby reducing or alleviating the adverse effect of the expansion of the two ends of the valve stent to form a dog-bone structure.

In a preferred example, from the middle of the valve stent to the two ends of the valve stent, the wall thicknesses of the oblique rods located in different rows may increase linearly, but not limited thereto.

In a preferred example, the wall thicknesses of the oblique rods in the middle row of the interventional valve stent 104 may be 0.47 mm (a range of the wall thickness may be from 0.20 mm to 0.80 mm, and the wall thicknesses ranging from 0.47 mm to 0.50 mm may be the most preferred), and from the middle to the two ends, a change of the wall thickness of each row may be from 0.01 mm to 0.10 mm (0.02 mm may be the most preferred), and the initial thickness may be 0.50 mm, but not limited thereto.

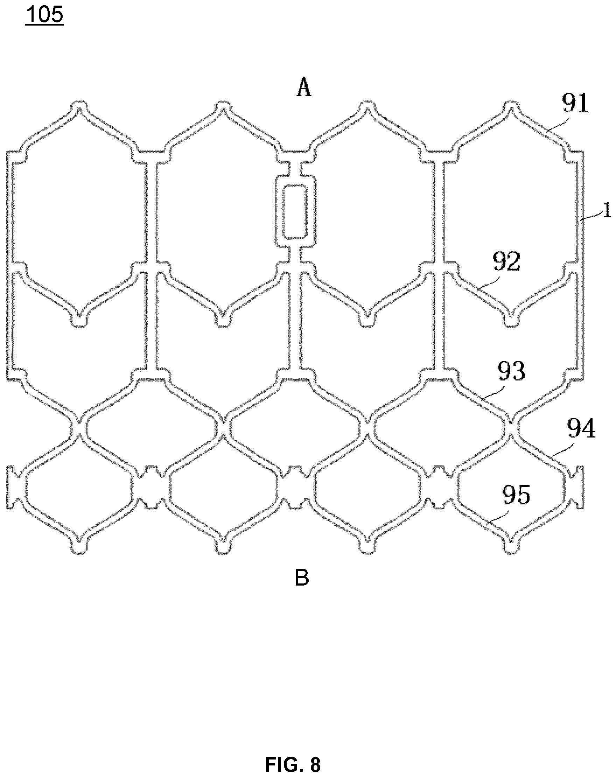

FIG. 8 is a flattened plan view illustrating a sixth type of interventional valve stent of the present disclosure. As shown in FIG. 8, embodiments of the present disclosure may also provide an interventional valve stent 105. The interventional valve stent 105 may include a valve stent defining a frame lumen. The valve stent may expand from a compressed state to an expanded state. The valve stent may include straight rods connecting an upstream port A and a downstream port B, and oblique rods connected between the straight rods. An upstream section, a midstream section, and a downstream section may be sequentially formed along a direction from the upstream port A to the downstream port B. An expansion strain provided by the oblique rods located in the midstream section to a circumferential direction of the valve stent may be greater than an expansion strain provided by the oblique rods located in the upstream section and the downstream section to the circumferential direction of the valve stent. When the valve stent expands, a rate difference between a rate of circumferential expansion of the midstream section and a rate of circumferential expansion of the upstream section, and a rate difference between the rate of circumferential expansion of the midstream section and a rate of circumferential expansion of the downstream section may be reduced. The straight rods may be distributed in parallel in a column direction. The oblique rods may be distributed between the straight rods in multiple rows along a row direction perpendicular to the column direction. From the upstream port A to the downstream port B, a first row of oblique rods 91, a second row of oblique rods 92, a third row of oblique rods 93, a fourth row of oblique rods 94, and a fifth row of oblique rods 95 may be distributed sequentially. The oblique rods may be connected in a V-shaped arrangement between two adjacent straight rods to form an included angle between rods. The oblique rods may satisfy a requirement: from a middle of the valve stent to two ends of the valve stent, rod widths, wall thicknesses, and included angles between rods of the oblique rods located in different rows all increase sequentially.

Obviously, the interventional valve stent 105 may have the structural features of the three interventional valve stents (the interventional valve stent 102 to the interventional valve stent 104: from the middle to the two ends, the gradual change of the rod width of oblique rods, the gradual change of the included angle between rods of the oblique rods, and the gradual change of the wall thickness of the oblique rods, which is not be repeated here) at the same time, which may effectively enhance the internal stress transmission of the oblique rods in the midstream section. By effectively accepting the stress transmitted from the oblique rods in the midstream section, the stress concentration of the oblique rods in the midstream section may be significantly reduced, so that the oblique rods in the midstream section may be more likely to be expanded under force, and the expansion rate of the oblique rods at both two ends can be achieved, thereby reducing or alleviating the adverse effect of the expansion of the two ends of the valve stent to form a dog-bone structure.

Embodiments of the present disclosure may also provide an aortic valve. The aortic valve may include the above-mentioned interventional valve stent, and a valve leaflet arranged in a frame lumen formed by the valve stent. The aortic valve may have structural features of any of the above-mentioned interventional valve stents, which is conducive to enhancing the internal stress transmission of the oblique rods in the midstream section. By effectively accepting the stress transmitted from the oblique rods in the midstream section, the stress concentration of the oblique rods in the midstream section may be reduced, so that the oblique rods in the midstream section may be more likely to be expanded under force, and the expansion rate of the oblique rods at both two ends can be achieved, thereby reducing or alleviating the adverse effect of the expansion of the two ends of the valve stent to form a dog-bone structure.

To sum up, the purpose of the present disclosure may be to provide an interventional valve stent and an aortic valve, which is conducive to enhancing the internal stress transmission of the oblique rods in the midstream section. By effectively accepting the stress transmitted from the oblique rods in the midstream section, the stress concentration of the oblique rods in the midstream section may be reduced, so that the oblique rods in the midstream section may be more likely to be expanded under force, and the expansion rate of the oblique rods at both two ends can be achieved, thereby reducing or alleviating the adverse effect of the expansion of the two ends of the valve stent to form a dog-bone structure.

The above content is a further detailed description of the present disclosure in combination with specific preferred embodiments, and it cannot be assumed that the specific implementation of the present disclosure is limited to these descriptions. For those of ordinary skill in the technical field of the present disclosure, without departing from the concept of the present disclosure, some simple deduction or replacement can also be made, which should be considered as belonging to the protection scope of the present disclosure.

## Claims

1. An interventional valve stent, comprising:
a valve stent defining a frame lumen, the valve stent including straight rods connecting an upstream port and a downstream port, and oblique rods connected between the straight rods;
an upstream section, a midstream section, and a downstream section being sequentially formed along a direction from the upstream port to the downstream port, when the valve stent expands from a compressed state to an expanded state, an expansion strain provided by the oblique rods located in the midstream section to a circumferential direction of the valve stent being greater than an expansion strain provided by the oblique rods located in the upstream section and/or the downstream section to the circumferential direction of the valve stent, to compensate for a rate difference between a rate of circumferential expansion of the midstream section, and a rate of circumferential expansion of the upstream section and/or a rate of circumferential expansion of the downstream section;
the straight rods being distributed in parallel in a column direction, and the oblique rods being distributed between the straight rods in multiple rows along a row direction perpendicular to the column direction;
the oblique rods being connected in a V-shaped arrangement between two adjacent straight rods to form an included angle between rods; and
the oblique rods satisfying a requirement: from a middle of the valve stent to two ends of the valve stent, rod widths, wall thicknesses, and included angles between rods of the oblique rods located in different rows all increase sequentially.

2. An interventional valve stent, comprising:
a valve stent defining a frame lumen, the valve stent including straight rods connecting an upstream port and a downstream port, and oblique rods connected between the straight rods;
an upstream section, a midstream section, and a downstream section being sequentially formed along a direction from the upstream port to the downstream port, when the valve stent expands from a compressed state to an expanded state, an expansion strain provided by the oblique rods located in the midstream section to a circumferential direction of the valve stent being greater than an expansion strain provided by the oblique rods located in the upstream section and/or the downstream section to the circumferential direction of the valve stent to compensate for a rate difference between a rate of circumferential expansion of the midstream section, and a rate of circumferential expansion of the upstream section and/or a rate of circumferential expansion of the downstream section.

3. The interventional valve stent of claim 2, wherein
during an expansion process of the valve stent, the rate of circumferential expansion of the midstream section is the same as the rate of circumferential expansion of the upstream section and/or the rate of circumferential expansion of the downstream section.

4. The interventional valve stent of claim 2, wherein
the straight rods are distributed in parallel in a column direction, and the oblique rods are distributed between the straight rods in multiple rows along a row direction perpendicular to the column direction.

5. The interventional valve stent of claim 2, wherein
from a middle of the valve stent to two ends of the valve stent, rod widths of the oblique rods located in different rows increase sequentially.

6. The interventional valve stent of claim 5, wherein
from the middle of the valve stent to the two ends of the valve stent, the rod widths of the oblique rods located in different rows increase linearly or in stages in an order of rows.

7. The interventional valve stent of claim 2, wherein
from a middle of the valve stent to two ends of the valve stent, wall thicknesses of the oblique rods located in different rows increase sequentially.

8. The interventional valve stent of claim 7, wherein
from the middle of the valve stent to the two ends of the valve stent, the wall thicknesses of the oblique rods located in different rows increase linearly or in stages in an order of rows.

9. The interventional valve stent of claim 2, wherein
the oblique rods are connected in a V-shaped arrangement between two adjacent straight rods to form an included angle between rods; and
from a middle of the valve stent to two ends of the valve stent, included angles between rods of the oblique rods located in different rows increase sequentially.

10. The interventional valve stent of claim 9, wherein
from the middle of the valve stent to the two ends of the valve stent, the included angles between rods of the oblique rods located in different rows increase linearly or in stages in an order of rows.

11. An aortic valve, comprising:
an interventional valve stent of any one of claims 1 to 10, and
a valve leaflet arranged in a frame lumen formed by the valve stent.
